Europäisches Patentamt

European Patent Office

Office européen des brevets

(11) Publication number: **0 310 639 B1**

# EUROPEAN PATENT SPECIFICATION

(45) Date of publication of patent specification: **03.03.93**

(51) Int. Cl.⁵: **A61K 31/195**, A61K 31/07, A61K 31/20, A61K 35/60, A61K 31/70

(21) Application number: **88902675.3**

(22) Date of filing: **19.02.88**

(86) International application number: **PCT/US88/00504**

(87) International publication number: **WO 88/06035 (25.08.88 88/19)**

The file contains technical information submitted after the application was filed and not included in this specification

(54) **OMEGA-3 FATTY ACIDS IN TRAUMATIC INJURY TREATMENT.**

(30) Priority: **20.02.87 US 17326**

(43) Date of publication of application:
**12.04.89 Bulletin 89/15**

(45) Publication of the grant of the patent:
**03.03.93 Bulletin 93/09**

(84) Designated Contracting States:
**AT BE CH DE FR GB IT LI LU NL SE**

(56) References cited:
**CA-A- 1 197 782
FR-A- 2 124 087
US-A- 4 401 657
US-A- 4 678 808**

**ALEXANDER ET AL, "The Importance of Lipid Type in the Diet after Burn Injury", Annals of Surgery Volume 204, Number 1, issued July 1986, see abstract, page 2 and page 7**

(73) Proprietor: **SHRINERS HOSPITALS FOR CRIP-PLED CHILDREN
Post Office Box 31356
Tampa, FL 33631-3356(US)**

(72) Inventor: **ALEXANDER, J., Wesley
2869 Grandin Road
Cincinnati, OH 45208(US)**

(74) Representative: **Smolders, Walter et al
c/o Sandoz AG Patentabteilung
CH-4002 Basel (CH)**

Chemical Abstracts, Vol.104, issued 1986(Columbus, Ohio, USA), EDWARD A. MASCIOLI ET AL, "Dietary Supplement and Method of Miminizing Effects of Infection through Diet", see page 364, column 2, abstract no. 230488k, PCT Int. Appl. WO8600523, 30 Jan. 1986

MOCHIZUKI ET AL, "Optimal Lipid Content for Enteral Diets following Thermal Injury", JPEN, Vol. 8, No. 6, issued 1984, see abstract, page 639, column 1, and Table III.

Information sheet concerning "Multivitamin Concentrate" from Lypho Med.(R)

## Description

Field of the Invention

This invention relates to a nutritionally fortified pharmaceutical composition for the enteral administration to patients in a hypermetabolic state such as those suffering from a substantial burn, trauma, or major surgery, and more particularly to a composition for enteral administration to patients who have encountered a substantial burn injury to the skin or other areas from contact with heat, radiation, electricity or chemicals.

Disclosure Statement

Patients suffering from a traumatic injury such as a substantial burn (involving an area greater than about 45 percent of the surface area of a human) become hypermetabolic and are highly susceptible to the development of malnutrition and infection. In 1970 of the patients who survived more than one week, roughly 75 percent died of infectious complications. While substantial improvement has taken place since then, what is needed is an improved nutritionally fortified pharmaceutical composition which will aid the traumatically injured patient manifesting or about to manifest a hypermetabolic state associated with a traumatic injury by attenuating the hypermetabolic state thereby lessening malnutrition and infection associated with a traumatic injury. In patients where the GI tract is still functioning but who are unable to orally take in adequate amounts of nutrients, enteral nutrition is the preferred route of nutritional administration relative the parenteral route.

Generally speaking, enteral nutrition products may be administered orally or by tube feeding routes. The nasogastric, nasoduodenal and nasojejunol routes are nonsurgical. Whereas, the jejunostomy, gastrostomy and esophagostomy are surgically inserted. Enteral nutrition products may also be administered by mouth where the patient is able. The nasoduodenal and nasojejunal are generally used in an unconscious patient and those with an impaired gag reflex in order to minimize the likelihood of aspiration.

Numerous enteral formulations are utilized in patients with a hypermetabolic state as effected by burns, trauma, major surgery and in those patients with malnutrition syndromes, neoplasms, chronic illnesses and in disorders resulting from prolonged periods of reduced oral intake resulting from cerebral vascular accidents or a comatose state.

ISOCAL® is an enteral formulation by Mead Johnson which utilizes casein and soy for its protein source, glucose oligosacchrides for its carbohydrate source and soy oil and medium chain triglycerides (MCT) oll for its lipid source. The composition includes about 19 grams linoleic acid per liter.

OSMOLITE® is manufactured by Ross and utilizes as its protein source casein and soy, corn starch for its carbohydrate source and fifty percent MCT oil, forty percent corn oil and ten percent soy oil for its lipid source. The composition includes about 11.5 grams linoleic acid per liter.

ENSURE® is manufactured by Ross and utilizes casein and soy for protein source, corn starch and sucrose for a carbohydrate source and corn oil for a lipid source. The composition includes about 19.6 grams linoleic acid per liter.

SUSTACAL® manufactured by Mead Johnson utilizes casein and soy for its protein source, corn syrup and sucrose for its carbohydrate source and soy oil for its lipid source. The composition includes about 6.8 grams linoleic acid per liter.

ENSURE PLUS® manufactured by Ross is a high protein composition using soy and casein for its protein source, corn starch and glucose for its carbohydrate source and corn oil for its lipid source. The composition includes about 27 grams linoleic acid per liter.

MAGNACAL® manufactured by Chesebrough Ponds is a high density composition with 2.0 calories/ml. MAGNACAL utilizes casein for its protein source, corn syrup for its carbohydrate source and soy oil for its lipid source. The composition includes about 59 grams linoleic acid per liter.

TRAUMACAL® manufactured by Mead Johnson utilizes casein for its protein source, corn syrup and sucrose for its carbohydrate source and 70 percent soy bean oil and 30 percent MCT oil for its lipid source. The composition includes about 27 grams linoleic acid per liter.

PRECISION ISOTEIN® HN is manufactured by Sandoz and utilizes lactalbumin for its protein source, maltodextrin for its carbohydrate source and soy oil and MCT oil for its lipid source. The composition includes about 3.4 grams linoleic acid per liter.

The above enteral compositions fail to provide omega-3 fatty acids including eicosapentaenoic acid or a nutritionally fortified pharmaceutical composition containing omega-3 fatty acids of fish oil including eicosapentaenoic acid for the reduction or attenuation of hypermetabolic states associated with traumatic injuries such as a burn injury, trauma and major surgery and especially substantial burn injuries.

Therefore, it is an object of this invention to provide a nutritionally fortified pharmaceutical enteral composition to aid in the treatment of a traumatic injury with an impaired immune response and an associated hypermetabolic state especially where the hypermetabolic state and immunologic depression are the result of a traumatic injury such as a substantial burn injury.

The enteral composition of the invention is designed to provide omega-3 fatty acids including eicosapentaenoic acid in an amount sufficient to reduce the hypermetabolic resting metabolic state associated with those suffering from a traumatic injury and to limit the amount of linoleic acid to the amount needed for preventing essential fatty acid deficiency;

This decreases the amount of arachidonic acid pathway metabolites formed in a traumatically injured patient thereby enhances healing of a traumatic injury in the patient in need of such treatment.

Accordingly, the composition of the invention is to provide to a patient suffering from a traumatic injury, such as a burn injury, omega-3 fatty acids including eicosapentaenoic acid in an amount sufficient to attenuate a hypermetabolic response associated with the traumatic injury and limiting the amount of linoleic acid to the amount needed for preventing essential fatty acid deficiency to enhance the healing rate of the burn injury.

## SUMMARY OF THE INVENTION

The nutritionally fortified pharmaceutical enteral composition of the present invention is defined by the appended claims with a specific embodiment shown in the included formulations and figures. For purposes of summarizing the invention, the invention relates to a composition suitable for treating a traumatic injury by improving immunologic response and reducing a hypermetabolic resting metabolic state associated with those suffering from the traumatic injury such as a substantial burn, trauma or major surgery.

Enteral support is the preferred route of nutrient delivery. However, diarrhea often accompanies this route, especially continuous nasogastricc hyperalimentation. Diarrhea disturbs fluid and electrolyte balance and worsens nutritional status. Tube feeding hyperosmolality and serum albumin levels are the suggested key causative factors in tube feeding-induced diarrhea via hypertonicity mechanisms.

The low fat, Vitamin A containing composition according to the invention is suitable for the treatment of a traumatic injury and decreases the risk of diarrhea.

It is preferably enterally administered within 48 hours after the inflection of the traumatic injury and most preferably within 24 hours. The enteral composition comprises:

an intact protein, in an amount of from 20 to 30 percent of the total energy intake;

carbohydrate, in an amount of from 65 to 70 percent of the total energy intake;

Vitamin A in an amount sufficient to provide from 5,000 I.U. to 50,000 I.U. per day;

lipids, in an amount of from 7 to 15 percent of the total energy intake in the form of a mixture of fish oil and safflower oil comprising linoleic acid in an amount of 3 to 4 % of the total energy intake and omega-3 fatty acids of fish oil including eicosapentaenoic acid in an amount sufficient to reduce a hypermetabolic resting metabolic state associated with one suffering from a traumatic injury, especially a substantial burn injury.

The enteral composition which substantially decreases the risk of diarrhea usually associated with tube feeding formulas, includes an amount of Vitamin A sufficient to provide 5,000 I.U. per day for enteral administration to a child from 9 months through 3 years of age. For a patient older than 3 years, including an adult, the enteral composition includes an amount of Vitamin A greater than about 10,000 I.U. per day. Possible toxic effects of large doses of Vitamin A exist, however amounts up to 50,000 I.U. daily are considered safe for up to 8 months. Preferably the enteral composition utilizes whey for the intact protein, a mixture of substantially equal amounts of nonprotein calories provided by fish oil and by safflower oil, where the lipid supplies about 15 percent of the nonprotein calories for the lipid and includes arginine, supplying about 2 percent of the total energy intake, and cysteine and histidine each supplying about one-half percent of the total energy intake.

The intact protein may be derived from lactalbumin, egg albumen or whey,. A property common to the protein which may be used in the invention is that it have a high biologic value. That is, that the protein is better at supporting animal growth than a protein with a low biologic value. The intact protein supplies from 20 to 30 percent of the total energy intake of the patient. Where the total energy intake of protein is greater than 30 percent, adverse effects, such as weight loss, appear. The optimum range appears to be 20-25 percent of the total energy intake. The preferred intact protein is whey protein.

The carbohydrate is selected from intact carbohydrates, complex glucose polymers and disaccharides. The intact form of a carbohydrate is derived from cereals, pureed vegetables and complex starches. The intact carbohydrate may be partially hydralized to yield complex glucose polymers. The preferred carbohydrate is a complex glucose polymers such as POLYCOSE® (Ross Laboratories, Columbus, Ohio) or SUMACAL® (Chesebrough-Ponds).

The elemental forms of either carbohydrate and/or protein require less digestive capability than the intact macronutrients such as the intact protein or intact carbohydrate. However, the increased number of particles with the elemental formulations will increase osmolality which may potentially increase certain adverse effects such a diarrhea.

The composition of the invention preferably includes nutritionally necessary expedients such as vitamins and minerals. Commercial preparations such as NUTRISOURCE® Vitamins and NUTRISOURCE® minerals by Sandoz are readily available.

The lipid provides 7-15 percent of the total calories and always includes linoleic acid in an amount of 3 to 4 % of the total energy intake to prevent an essential fatty acid deficiency thereof. The lipid also always includes omega-3 fatty acids of fish oil including eicosapentaenoic acid in an amount sufficient to attenuate or reduce a hypermetabolic resting metabolic state resulting from a traumatic injury, especially a substantial burn injury. Any remaining lipid which is needed to attain the 7-15 percent of the total calories is provided by lipids which do not enter the arachidonic acid pathway.

The remaining lipid of the inventive composition may be provided by long-chain fatty acids which are present in butterfat and vegetable oils. The vegetable oils include peanut oil, olive oil, safflower oil and sunflower oil. The amount of linoleic acid in vegetable oils must be considered when determining the amount to be used in the composition without providing any excess over that required to prevent a deficiency thereof. The lipid may be further provided by medium chain triglycerides (MCT). When medium chain triglycerides are used an effective amount of essential fatty acids must be either included in the composition of the invention or otherwise provided to the patient in order to prevent essential fatry acid deficiency. Preferably, the omega-3 fatty acids including eicosapentaenoic acid are provided by fish oil. Omega-3 fatty acids C20:5 eicosapentaenoic acid and C22:6 docosahexaenoic acid are typical of fish oil. More specifically, the omega-3 fatty acids of fish oil include eicosapentaenoic acid, docosahexaenoic aid, docosapentaenoic acid and linolenic acid. The exact percentages of fatty acids in fish oil may vary between species.

The fatty acid components of fish oil include:

C14:0 (Myristic) 7.01%; C14:1 - 0.36%;
C15:0 - 0.63%; C15:1 and C15:0 (BR) - 0.14%;
C16:0 - 17.03%; C16:1 (palmitoleic) - 8.68%;
C17:0 - 2.24%; C17:1 and C17:0 (BR) - 1.11%;
C18:0 (Stearic) - 4.79%; C18:1 (oleic) - 12.75%;
C18:2 (linoleic) - omega 6 - 1.79%; 18:3 (linolenic) - omega 3 - 0.46%;
C20:0 (arachidic) - 2.19%;
C20:1 - 1.57%; C20:5 (eicosapentaenoic) - omega-3 - 17.43%;
C22:1 - 0.73%;
C22:5 (docosapentaenoic) - omega-3 - 3.20%;
C22:6 (docosahexaenoic) - omega-3 - 16.84%.

See Sanders, T.A.B. and Roshanai, F., Clinical Science, Vol. 64 pp. 91-99, 1983 Cod liver oil also contains omega-3 fatty acids. However, because of the amount necessary to provide sufficient omega-3 fatty acids, toxicity from high levels of Vitamin A may arise. Therefore, the use of cod liver oil as a source of omega-3 fatty acids is less desirable. Further, any arachidonic acid present in any oil would may have to be considered relative the amount of linoleic acid since arachidonic acid is derived from linoleic acid and enters the arachidonic acid pathway. A mechanical mixture of the omega-3 fatty acids as present in fish oil would be expected to perform substantially as fish oil which naturally includes omega-3 fatty acids.

In a preferred embodiment, the lipid consists of a mixture of substantially equal amounts of nonprotein calories provided by fish oil which contains eicosapentaenoic acid and by safflower oil, where the lipid supplies about 15 percent of the nonprotein calories.

Safflower oil, commercially available as MICROLIPID® (Organon Inc., West Orange, N.J.) contains about 74 percent linoleic acid. Fish oil and marine oils contain high levels of eicosapentaenoic acid. MaxEPA® (R.P. Scherer Corp., Clearwater, FL) is a commercially available fish oil which contains about 18 percent eicosapentaenoic acid.

The traumatic injury is treated by administering an amount of the enteral composition according to the invention sufficient to reduce or inhibit a hypermetabolic resting metabolic state associated with those suffering from a traumatic injury such as a substantial burn, trauma or major surgery and especially to those suffering from a substantial thermal burn injury to the skin or other areas.

Preferably, the composition is administered to one suffering from traumatic injury as soon as possible after the injury to maximize the benefit of inhibiting or attenuating the hypermetabolic response and to lessen the risk of infection by improving immunologic response. Increases in resting energy expenditure and catabolic hormones are reduced when enteral feeding is implemented immediately after a burn. Conversely, when enteral restriction is imposed while passively waiting for post-traumatic ileus to resolve, a noticeable hypermetabolic syndrome follows. Typically, metabolic rate is normal on the first two days following burn injury and then climbs dramatically over the course of several weeks. However, when burns exceed about 50 percent total body surface area or in some cases of sepsis, metabolic rate plateaus or may even decrease. Evidence appears to support a chronic elevation of catecholamines which act as the dominant stimulus of the hypermetabolic response to burns. Increased plasma catecholamines and high urinary catecholamine excretion have been correlated with burn size and metabolic rate. Enteral feeding should begin within 48 hours postburn and preferably within 24 hours postburn and most preferably within 6 hours postburn. The suppression of hypermetabolic response is evidenced by decreased resting energy expenditure, positive nitrogen balance and normal serum concentration of the catabolic hormones. Other benefits from early GI feeding include the ability to immediately satisfy nutritional requirements along with promoting bowel mucosal integrity and improved tube feeding tolerance. Preferably, the enteral composition is administered by utilizing a nasogastric tube connected to low Gomco suction while simultaneously enterally feeding via a nasoduodenal tube within 24 hours postburn.

Preferably the composition includes the omega-3 fatty acids of fish oil eicosapentaenoic and docosahexaenoic acids. Most preferably the composition includes fish oil containing the omega-3 fatty acids eicosapentaenoic acid and docosahexaenoic acid.

The continuous enteral administration of the composition of the invention is preferred over the intermittent enteral administration of the composition of the invention. The composition of the invention is administered to one in need as soon as possible after the infliction of the traumatic injury.

The composition of the invention is intended to be the sole source of dietary energy or total energy intake (protein, carbohydrate and lipid) for the time the patient is unable to take food by mouth. Typically, a patient who has suffered traumatic injury, especially a traumatic burn injury, is not able to take food by mouth for a period of time following the injury.

## BRIEF DESCRIPTION OF THE DRAWINGS

For a fuller understanding of the nature and objects of the invention, reference should be had to the following detailed description taken in connection with the accompanying drawings in which:

Fig. 1 is a comparison of the metabolic rates of different groups of animals receiving similar diets, except for the amino acid composition, by continuous administration;

Fig. 2 is a comparison of resting metabolic rates of burned animals receiving different types of lipids in their diets;

Fig. 3 is a simplified display of the metabolites of arachidonic acid; and

Fig. 4 is a comparison of the dienoic and trienoic prostaglandin pathways.

Similar reference characters refer to similar parts throughout the several views of the drawings.

## DETAILED DISCUSSION

The present invention discloses a nutritionally fortified pharmaceutical composition which is suitable for using in patients suffering from traumatic injuries, such as burn injuries which initiate a hypermetabolic state.

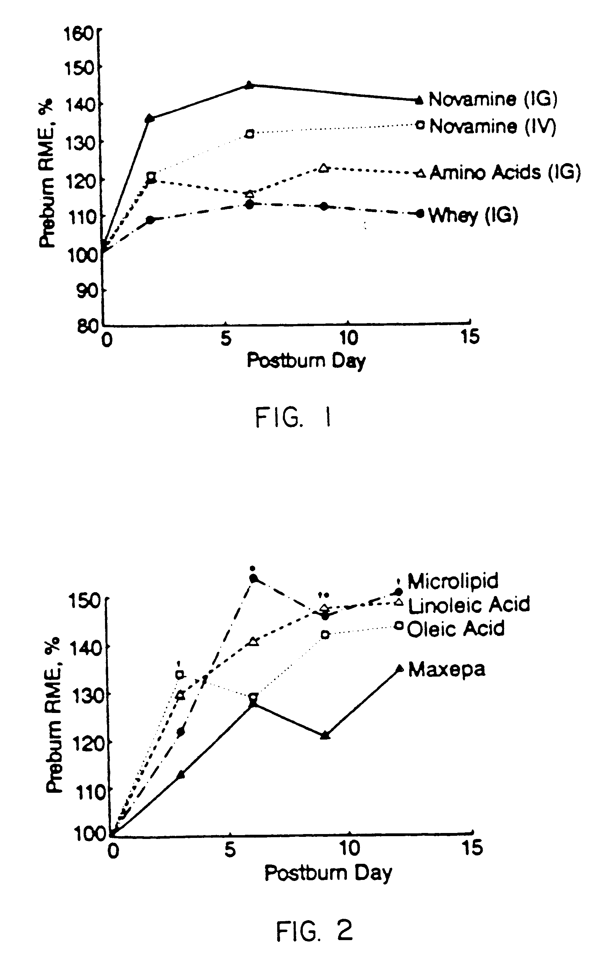

Fig. 1 is a comparison of the resting metabolic expenditure (RME) of different groups of animals continuously receiving similar diets (isocaloric, isonitrogenous and isovolemic) except for the amino acid composition. NOVAMINE®, a commercially available amino acid composition was administered intragastrically (IG) and intravenously (IV). An Amino Acid group was administered intragastrically (IG) and comprised crystalline amino acids in the same amounts found in whey protein. As illustrated at Fig. 1, all groups demonstrated an increase in RME, however, the group receiving whey protein clearly demonstrated whey protein's ability to keep the resting metabolism nearest to the preburn RME. The crystalline amino acids present in the same amounts as found in whey protein also restricted the metabolic rate increase, but not to the extent of the intact whey protein.

Fig. 2 is a comparison of resting metabolic rates of burned animals receiving similar diets but different types of lipids: MICROLIPID® (74 percent linoleic acid and 15 percent oleic acid), linoleic acid, oleic acid and MaxEPA®. The lipid comprised 10 percent of the total energy intake. As clearly indicated the hypermetabolic response was inhibited for the group of animals which received MaxEPA® (fish oil). It is theorized that linoleic acid and other omega-6 fatty acids enter the arachidonic acid pathway thereby increasing the production of certain metabolites (dienoic prostaglandins) which appear to have adverse effects on the healing process of one suffering from a traumatic injury, such as a substantial burn. Among the metabolites are the dienoic prostaglandins $PGE_2$, $PGI_2$ and $TXA_2$. Fig. 2 illustrates that arachidonic acid pathway metabolites contribute to the adverse effects of lipids following burn injury, as evidenced by the differences in inhibition of the hypermetabolic state by the compared lipids.

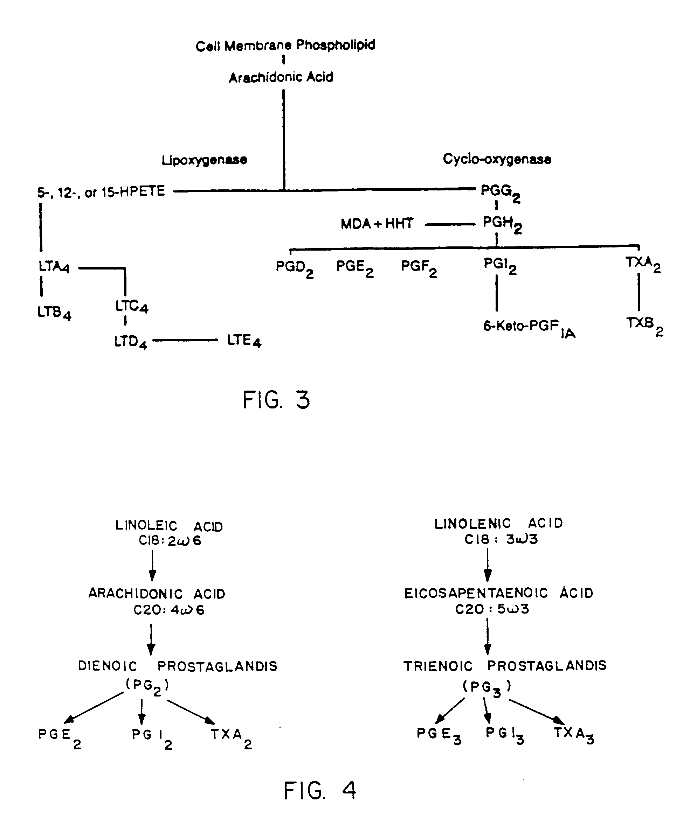

Fig. 3 is a simplified display of the metabolites of arachidonic acid. HPETE indicates 5(S)-hydroperoxy-6,8,14-eicosatetraenoic; LT = leukotriene; PG = prostaglandin; MDA = malondialdehyde; HHt = (12S)-12-hydroxy-5,8,10-hepatodecatrienoic and TX = thromboxane. It appears that the balance between $TXA_2$ and $PGI_2$ takes part in the regulation of vascular responses, platelet aggregation and thrombosis. $PGE_2$ by itself is a vasodialtory prostaglandin that may cause edema formation. However, $PGE_2$ in combination with bradykinin, histamine, and/or degradation products of a complement pathway present a synergistic potentiation in the formation of edema. More importantly, $PGE_2$ is a potent depressant of immune response and has feedback mechanisms that inhibit the activities of interleukin-1 and interleukin-2 as well as stimulating the development and activity of suppressor T cells. Accordingly, the biological effects manifested by the metabolites of the arachidonic acid pathway explains the adverse effects manifested by their presence.

Fig. 4 is a comparison of the dienoic and trienoic prostaglandin pathways. TX = thromboxane. The trienoid prostaglandin metabolites $PGI_3$, $PGE_3$ and $TXA_3$ are equivalent to the major metabolites $PGE_2$, $PCI_2$ and $TXA_2$ of the dienoid prostaglandins. However, $PGE_3$ and $TXA_3$ lack potency when compared with their dienoid counterparts and block the action of $PGE_2$ and $TXA_2$. Thus, regulation of dietary lipids by controlling the intake of omega-6 fatty acids controls the synthesis of dienoic prostaglandins and therefor the adverse effects associated therewith.

A more through discussion is set forth in: Alexander, J. Wesley, Nutrition and Infection, Arch. Surg., Volume 21, pp. 966-972 (August 1986); Alexander, J. Wesley, The Importance of Lipid Type in the Diet after Burn Injury, Ann. Surg., Volume 204, No. 1, pp. 1-8 (July 1986) and, Gottschlich, Michele and Alexander, J. Wesley, Fat Kinetics and Recommended Dietary Intake in Burns, J. Parenteral and Enteral Nutrition, Volume 11, No. 1, pp. 80-85 (1987), and Trocki O., Saito H., Gonce S.J., Heyd T.J., Alexander J.W., Effects of Dietary Lipids on Postburn Metabolic and Immune Response, JPEN 10:50, 1986

Fish oil which is rich in trienoic prostaglandin precursors exerts beneficial effects on gastrointestinal, immunologic and inflammatory response when compared to fat from vegetable oils which are largely composed of dienoic prostaglandin precursors in the management of burns.

In one embodiment of the invention an enteral composition utilized as its protein source, 87 percent whey protein with 9 percent arginine, 2 percent cysteine and 2 percent histidine (100 percent of the protein) at 57 grams per liter. The protein source supplied about 20 percent of the total energy intake required by the patient. The carbohydrate source was maltodextrin which supplied about 85 percent of the nonprotein calories (about 68 percent of the total energy intake) and was added at a rate of 164 grams per liter. The lipid source consisted of fish oil and safflower oil providing 15 percent of the nonprotein calories (about 12 percent of the total calories) with substantially equal amounts of nonprotein calories provided by the fish oil (MaxEPA®), which contained eicosapentaenoic acid and docosahexaenoic acid, and by the safflower oil. The fish oil-safflower mixture was added at a rate of 13 grams per liter. The fish oil-safflower oil mixture included 4.8 grams linoleic acid per liter. The composition of the invention supplies about 1000 kcalories per liter and had an osmolality of 549 mOsm/Kg water.

An enteral composition of the invention was prepared as set forth below:

| Modular Tube Feeding-Shriners Burns Institute | | | | | |
|---|---|---|---|---|---|
| Ingredients | Amount | CHO | PRO | FAT | KCAL |
| Sterile Water | 750 ml | | | | |
| MaxEPA (fish oil) | 6 ml | 0 | 0 | 6 | 54 |
| MICROLIPID | 9 ml | 0 | 0 | 4.4 | 39 |
| PROMIX | 62 g | 3.3 | 50 | 2.6 | 236 |
| SUMACAL | 149 g | 142 | 0 | 0 | 568 |
| Arginine HCL | 5 g | 0 | 5 | 0 | 20 |
| Histidine | 1 g | 0 | 1 | 0 | 4 |
| Cysteine | 1 g | 0 | 1 | 0 | 4 |
| NUTRISOURCE Minerals | 24 g | 6 | 0 | 0 | 24 |
| NUTRISOURCE Vitamins | 20 g | 18 | 0 | 0 | 72 |
| Vitamin A (50,000 units/ml) | 0.1 ml | 0 | 0 | 0 | 0 |
| TOTAL | | 163.3 | 57 | 13 | 1021 |

The sterile water was measured and poured into a blender, such as a Waring blender. The MaxEPA® (R. P. Scherer Corporation) (fish oil) was measured using a pipette and/or graduated cylinder. The MICROLIPID® (Chesebrough-Ponds) preparation was vigorously shaken and measured utilizing a pipette. Both of these liquids are added to the blender. PROMIX® (Navaco) was weighed out and added to the blender. SUMACAL® (Chesebrough-Ponds) was weighed and add to liquids in blender. The Arginine HCL, Histidine and Cysteine were individually weighed and added to the liquids in the blender. Twenty four grams of NUTRISOURCES® (Sandoz) Minerals and 20 grams of NUTRISOURCE® (Sandoz) Vitamins were added to the liquids in the blender. Vitamin A was measured and added to the liquids in the blender. All ingredients were mixed in the blender at low speed for about 30 seconds taking caution not to over mix. The foam was allowed to settle. To check proper recipe level, the liquid was poured into a 2000 ml flask and more sterile water was added if necessary to bring up to the correct 1000 ml level. The formula provided about 1000 Kcal. The formula was transferred into brown bottle and refrigerated. After 24 hours any remaining formula is discarded. To prepare an cnteral composition of the invention which decreases the risk of diarrhea, an amount of Vitamin A is added to the composition as described above.

CALCULATION FORMULA FOR 1000 ml

Kcal needs = 1000
Protein needs (20%) of Kcal) = 200 Kcal or 50 g protein
Arginine needs (2% of Kcal) = 20 Kcal or 5 g arginine
Histidine needs (0.5% of Kcal) = 5 Kcal or 1 g
Cysteine needs (0.5% of Kcal) = 5 Kcal or 1 g

$$\begin{array}{l} \text{1000 Kcal} \\ \underline{\text{-230 Kcal (arginine + histidine + cysteine + Promix®)}} \\ \text{770 Kcal from nonprotein sources} \end{array}$$

| | |
|---|---|
| Total fat | = 15% of nonprotein calories |
| | = .15 x 770 = 115 Kcal or 13 g fat |
| Fat from Maxepa® | = 7% of nonprotein calories |
| | = .07 x 770 = 54 Kcal or 6 g Maxepa® |
| | = 6 ml |
| Fat from Microlipid | = 8% of nonprotein calories |
| | = 13 - (2.6 + 6) |
| | = 4.4 g of Microlipid® |
| | = 9 ml |
| Carbohydrate | = 85% of nonprotein calories |
| | = .85 x 770 = 655 Kcal or 163 g carbohydrates 230 Kcal (protein) + 115 Kcal (fat) + 655 Kcal (carbohydrate) = 1000 Kcal |

The composition may further include about 220mg/day zinc sulfate where the occurrence of a suboptimal serum level is encountered in a traumatically injured patient. The composition may further include about 1 gm of vitamin C per day.

Diarrhea is a frequent problem in the use of enterally administered enteral compositions. In an effort to determine the cause, a study was conducted. Diarrhea was defined as greater than 4 liquid bowel movements per day or a large (greater than 200 g) liquid stool. Of the 20 patients studied, 6 (30%) developed diarrhea. Stool cultures were negative for pathogenic organisms. The mean total body surface area burn of those who developed diarrhea was 55 percent (range 12-89%) whereas those without diarrhea had a mean burn size of 43 percent (range 28-71%) (not significant). Results demonstrated a significant relationship between dietary lipid content and diarrhea. Implementation of tube feeding within 48 hours postburn was associated with a decreased incidence of diarrhea. The risk of diarrhea was most closely associated with inadequate Vitamin A intake. Surprisingly, tube feeding osmolality, systemic antibiotics, or hypoalbuminemia did not have an adverse effect on intestinal absorption. Various enteral feeding products were compared. This study demonstrated a highly significant effect of the lowfat enteral composition of the invention augmented with vitamin A, as set forth above, in decreasing the risk/incidence of diarrhea associated with enteral feeding.

Numerous methods and formulas exist for determining the daily caloric needs of traumatically injured patients. Formulas such as the Curreri formula has been found workable in initially projecting energy needs in burn patients: (25 kcal x kg body weight) + (40 kcal x percent burn). While helpful in estimating peak energy needs, the Curreri formula leads to overfeeding as the patient progresses. The failure of the Curreri formula and other conventional formulas, is that they do not account for many effectors of energy balance over time. The object of any formula must be to provide adequate calories for positive nitrogen balance and satisfactory maintenance of body weight without overfeeding. To attain this object, energy expenditure is ascertained by measuring oxygen consumption of the patient by means known in the art. However, other energy requiring activities such as physical therapy, hydrotherapy and dressing changes can elevate energy needs and must be considered. Therefore, providing nutritional intake equivalent to 1.3 times the measured energy expenditure at rest is associated with conditions of positive nitrogen balance and satisfactory maintenance of body weight in the majority of burn patients.

The composition may be prepared in a dry form, with the lipid in an accompanying vial, for subsequent reconstitution by the addition of water and lipid. Such methods are well known in the art. Also, the composition may be prepared as needed or prepared as a ready-to-use composition by methods well known in the art.

**Claims**

1.  An enteral composition comprising:

    an intact protein, in an amount of from 20 to 30 percent of the total energy intake;

    carbohydrate, in an amount of from 65 to 70 percent of the total energy intake;

    Vitamin A in an amount sufficient to provide from 5,000 I.U. to 50,000 I.U. per day;

    lipids, in an amount of from 7 to 15 percent of the total energy intake in the form of a mixture of fish oil and safflower oil comprising linoleic acid in an amount of 3 to 4 % of the total energy intake and omega-3 fatty acids of fish oil including eicosapentaenoic acid in an amount sufficient to reduce a hypermetabolic resting metabolic state associated with one suffering from a traumatic injury.

2.  The composition of Claim 1 including an amount of Vitamin A sufficient to provide more than 10,000 I.U. per day.

3.  The composition of Claims 1 or 2, wherein the traumatic injury is a substantial burn.

4.  The composition of Claims 1 to 3, wherein the intact protein is whey, the lipid is a mixture of substantially equal amounts of nonprotein calories provided by fish oil and by safflower oil, where the lipid supplies about 15 percent of the nonprotein calories and further includes arginine, supplying about 2 percent of the total energy intake, cysteine supplying about one-half percent of the total energy intake and and histidine supplying about one-half percent of the total energy intake.

9

**5.** A composition according to anyone of Claims 1 to 4, for use as a therapeutic nutritional product.


**Patentansprüche**

**1.** Enterale Zusammensetzung aus

einem intakten Protein in einer Menge von 20 bis 30% der gesamten Energieaufnahme,

Kohlenhydraten in einer Menge von 65 bis 70% der gesamten Energieaufnahme,

Vitamin A in einer solchen Menge, daß sich 5 000 IE bis 50 000 IE pro Tag ergeben, und

Lipiden in einer Menge von 7 bis 15% der gesamten Energieaufnahme in Form eines Gemisches aus Fischöl und Safloröl, das Linolsäure in einer Menge von 3 bis 4% der gesamten Energieaufnahme und omega-3-Fettsäuren von Fischöl unter Einschluß von Eicosapentaensäure in einer solchen Menge enthält, daß sich eine Erniedrigung eines hypermetabolisch bleibenden metabolischen Zustands bei einem Patienten ergibt, der an einem traumatischen Schaden leidet.

**2.** Zusammensetzung nach Anspruch 1, die eine solche Menge an Vitamin A enthält, daß sich mehr als 10 000 IE pro Tag ergeben.

**3.** Zusammensetzung nach Anspruch 1 oder 2, worin der traumatische Schaden eine starke Verbrennung ist.

**4.** Zusammensetzung nach Anspruch 1 bis 3, worin das intakte Protein Molke ist, das Lipid ein Gemisch aus im wesentlichen gleichen Mengen an Nichtproteinkalorien, die von Fischöl und von Safloröl stammen, ist, wobei das Lipid etwa 15% der Nichtproteinkalorien liefert, und wobei ferner Arginin in einer Menge von etwa 2% der gesamten Energieaufnahme, Cystein in einer Menge von etwa 0,5% der gesamten Energieaufnahme und Histidin in einer Menge von etwa 0,5% der gesamten Energieaufnahme vorhanden sind.

**5.** Zusammensetzung nach einem der Ansprüche 1 bis 4 zur Verwendung als ein therapeutisches nutritionales Produkt.


**Revendications**

**1.** Une composition entérale comprenant:
   - une protéine intacte, en une quantité représentant de 20 à 30% de l'apport énergétique total,
   - un glucide, en une quantité représentant de 65 à 70% de l'apport énergétique total,
   - de la vitamine A en une quantité suffisante pour fournir de 5000 UI à 50 000 UI par jour,
   - des lipides, en une quantité représentant de 7 à 15% de l'apport énergétique total sous forme d'un mélange d'huile de poisson et d'huile de carthame comprenant de l'acide linoléique en une quantité représentant de 3 à 4% de l'apport énergétique total et des acides gras oméga-3 de l'huile de poisson comprenant l'acide eicosapentaénoïque en une quantité suffisante pour réduire un état hypermétabolique au repos chez un patient souffrant d'une lésion traumatique.

**2.** La composition de la revendication 1 comprenant une quantité de vitamine A suffisante pour fournir plus de 10 000 UI par jour.

**3.** La composition de la revendication 1 ou 2, dans laquelle la lésion traumatique est une brûlure substantielle.

**4.** La composition des revendications 1 à 3, dans laquelle la protéine intacte est du lactosérum, le lipide est un mélange en quantités substantiellement égales de calories non protéiniques fournies par l'huile de poisson et par l'huile de carthame, le lipide fournissant environ 15% des calories non protéiniques, et qui comprend également de l'arginine fournissant environ 2% de l'apport énergétique total, de la cystéine fournissant environ 0,5% de l'apport énergétique total et de l'histidine fournissant environ 0,5% de l'apport énergétique total.

5.  Une composition selon l'une quelconque des revendications 1 à 4, pour l'utilisation comme produit nutritionnel thérapeutique.

FIG. 1

FIG. 2

**FIG. 3**

Cell Membrane Phospholipid

Arachidonic Acid

Lipoxygenase    Cyclo-oxygenase

5-, 12-, or 15-HPETE ——— PGG$_2$

MDA + HHT ——— PGH$_2$

LTA$_4$    PGD$_2$    PGE$_2$    PGF$_2$    PGI$_2$    TXA$_2$

LTB$_4$    LTC$_4$    6-Keto-PGF$_{1A}$    TXB$_2$

LTD$_4$ ——— LTE$_4$

**FIG. 4**

LINOLEIC ACID
C18:2ω6

ARACHIDONIC ACID
C20:4ω6

DIENOIC PROSTAGLANDIS
(PG$_2$)

PGE$_2$    PGI$_2$    TXA$_2$

LINOLENIC ACID
C18:3ω3

EICOSAPENTAENOIC ACID
C20:5ω3

TRIENOIC PROSTAGLANDIS
(PG$_3$)

PGE$_3$    PGI$_3$    TXA$_3$

13